# EUROPÄISCHE PATENTSCHRIFT

(11) **EP 0 895 465 B1**
(45) Veröffentlichungstag und Bekanntmachung des Hinweises auf die Patenterteilung: **13.10.1999**
(21) Anmeldenummer: 96912028.6
(22) Anmeldetag: 22.04.1996
(51) Int. Cl.: A61F 5/455

(54) **URINALTÜTE UND VERPACKUNG HIERFÜR**
URINAL CONE AND PACKAGING THEREFOR
CONE URINAIRE ET SON EMBALLAGE

(43) Veröffentlichungstag der Anmeldung: 10.02.1999
(73) Patentinhaber: Kaus, Reinhold, 65439 Flörsheim (DE)
(72) Erfinder: Kaus, Reinhold, 65439 Flörsheim (DE)
(74) Vertreter: Aue, Hans-Peter, Dipl-Ing.
(86) Internationale Anmeldenummer: EP9601674
(87) Internationale Veröffentlichungsnummer: WO9739706

(56) Entgegenhaltungen:
- EP-A- 0 158 602
- EP-A- 0 226 277
- WO-A-93/11691
- US-A- 4 023 216

## Beschreibung

Die Erfindung betrifft eine Urinaltüte, insbesondere für Personen weiblichen Geschlechts, zum Anlegen am Genitalbereich der Person zum Zwecke des Urinierens, die aus einer im kollabierten Zustand im wesentlichen dreieckigen Form in ein trichterartiges Gebilde mit einem Mantel mit einer oberen Öffnung großen Durchmessers und eine dieser gegenüberliegende unteren Öffnung kleinen Durchmessers aufrichtbar ist.

Bei ungünstigen Umgebungsbedingungen und -verhältnissen bereitet es oftmals insbesondere weiblichen Personen Probleme, zu urinieren. Häufig treten solche ungünstigen Umgebungsbedingungen und -verhältnisse dann auf, wenn eine Vielzahl von Personen zu irgendwelchen beliebigen Anlässen zusammentreffen. Dabei werden Toiletten, Toilettenanlagen oder toilettenähnliche Anlagen bereits schon nach weniger häufiger Benutzung derart verschmutzt, daß nachfolgende Personen durch Ekelgefühl abgehalten werden, ihre Notdurft auf solchen Toiletten zu verrichten. Das trifft zwangsläufig insbesondere auf weibliche Personen zu, da diese das Urinieren im Sitzen auf den Toiletten ausführen müssen. Solche Toilettenverschmutzungen können beispielsweise bei langzeitig zu benutzenden Verkehrsmitteln, wie Reisebussen, Eisenbahnzügen usw. auftreten.

Weitere ungünstige Umgebungsbedingungen und -verhältnisse ergeben sich z.B. in Ländern anderer Kulturen, in denen anstelle von Toilettenbecken, beispielsweise Abflußrinnen bzw. Abflußlöcher üblich sind. Diese Art der Toilettenbenutzung ist für Personen aus Ländern, in denen die Benutzung von Toilettenbecken üblich ist, ungewohnt und gewöhnungsbedürftig. Häufig ist die Benutzung derartiger Toilettenanlagen auch mit einem Verspritzen der abgelassenen Urinflüssigkeit verbunden. Vielfach lassen Toilettenanlagen allgemein hygienisch zumutbare Bedingungen vermissen.

Aus der US-A-4023216 ist eine Urinaltüte bekannt, die im kollabierten Zustand eine Vieleckform aufweist. An einem Ende der Urinaltüte ist eine schräg geschnittene Öffnung kleinen Durchmessers ausgebildet. Am entgegengesetzten Ende der Urinaltüte ist eine Öffnung großen Durchmessers ausgebildet, die von einem Klebefalz am Mantel weggerichtete geradlinige Abschnitte aufweist, die in kurze, gleichmäßig verlaufende Krümmungsabschnitte übergehen, welche wiederum in kurze, geradlinige Abschnitte übergehen, die durch einen ebenfalls geradlinigen Abschnitt verbunden sind. Die obere Öffnung großen Durchmessers der Urinaltüte ist sonach durch eine Vielzahl von unterschiedlich verlaufenden Kanten geprägt, die eine Vielzahl von Eckpunkten bilden.

Die EP-A-0226277 zeigt eine Urinaltüte, die im kollabierten Zustand eine im wesentlichen Dreieckform aufweist und eine untere Öffnung kleinen Durchmessers und eine obere Öffnung großen Durchmessers aufweist. Die Öffnung großen Durchmessers ist extrem schräg zu den Seitenkanten der Urinaltüte geschnitten und weist eine etwa geradlinige Schnittkante auf.

Die WO 93/11691 offenbart eine Urinaltüte, die eine im kollabierten Zustand dreieckige Form aufweist und mit einer unteren Öffnung kleinen Durchmessers und eine oberen Öffnung großen Durchmessers versehen ist. Die obere Öffnung großen Durchmessers besitzt im kollabierten Zustand einen sich im Radius im wesentlichen nicht verändernden Krümmungsverlauf.

Es ist daher Aufgabe der Erfindung, eine Urinaltüte, insbesondere für Personen weiblichen Geschlechts, zum Anlegen am Genitalbereich der Person zum Zwecke des Urinierens zu schaffen, die den Personen das hygienische Urinieren unter unhygienischen oder ungünstigen Bedingungen ermöglichen soll und die darüber hinaus leicht verfügbar ist, einfach gehandhabt und nach Gebrauch entsorgt werden kann.

Erfindungsgemäß wird die Aufgabe dadurch gelöst, daß die Außenkontur des Mantels im kollabierten Zustand der Urinaltüte im Bereich der oberen Öffnung großen Durchmessers einen sich von einer Seite zur anderen Seite der im wesentlichen dreiekkigen Form des Mantels, die sich jeweils von der unteren Öffnung kleinen Durchmessers erstrecken, kontinuierlich verjüngenden Krümmungsradius aufweist, wobei die obere Öffnung des Mantels derart im Genitalbereich der Person anlegbar ist, daß dieser Bereich dicht umschlossen wird. Die Urinaltüte wird aus ihrem flachen Zustand einfach in ein trichterartiges Gebilde aufgerichtet. Mit ihrer oberen Öffnung wird diese am Genitalbereich der Person, beispielsweise um die Vagina einer weiblichen Person, angelegt, so daß die Öffnung kleinen Durchmessers nach unten gerichtet ist und beim Urinieren einen dünnen Urinstrahl erzeugt, der z.B. in eine Toilettenanlage abgeleitet wird. Nach dem Gebrauch der Urinaltüte wird diese einfach beispielsweise zusammengefaltet in einem Abfallkorb entsorgt. Die Benutzung der Urinaltüte erlaubt es der jeweiligen Person im Stehen, im Sitzen oder in hockender Stellung zu urinieren, ohne daß diese in Berührung mit der Toilettenanlage kommt. Durch den mittels der unteren Öffnung kleinen Durchmessers gebildeten Urinstrahl wird ein Verspritzen der Urinflüssigkeit beim Austreten aus der Urinaltüte vermieden.

Durch den sich kontinuierlich verjüngenden Krümmungsradius der oberen Öffnung großen Durchmessers der Urinaltüte wird gewährleistet, daß die Urinaltüte beim Aufrichten aus ihrem kollabierten Zustand an die Körperform im Genitalbereich der benutzenden Person, deren Genitalbereich dicht umschließend, angepaßt werden kann. Es bleibt hierbei dem Benutzer überlassen, selbst die beste Anlageform für die Urinaltüte an seinen Körper zu finden.

Nach einer Weiterbildung der Erfindung ist in deren kollabiertem Zustand die untere Öffnung kleinen Durchmessers abgeschrägt. Durch diese Maßnahme wird der Urinstrahl im Querschnitt derart verändert, daß bei dessen Auftreffen auf dem Boden ein Verspritzen der Urinflüssigkeit im wesentlichen vermieden wird.

Zweckmäßig ist es wenn die untere Öffnung einen Durchmesser von wenigstens 5 mm und die obere Öffnung einen Durchmesser von wenigstens 50 mm aufweist, um eine gute Funktionsfähigkeit der Urinaltüte zu gewährleisten.

Darüber hinaus weist die Urinaltüte einen die obere Öffnung großen Durchmessers und die untere Öffnung kleinen Durchmessers verbindenden Klebstreifen entlang des trichterförmigen Mantels auf. Der einteilig ausgebildete Mantel der Urinaltüte umfaßt diesen Klebstreifen, um diese in ein trichterförmiges Gebilde aufrichten zu können. Vorzugsweise ist der Klebstreifen am trichterförmigen Mantel verklebbar. Darüber hinaus kann daß der Mantel an zumindest einer seiner Seiten durch Rändeln oder dergleichen verbunden sein.

Um die unbenutzte Urinaltüte z.B. in einer Handtasche transportieren zu können, wobei diese möglichst wenig Platz beanspruchen soll, ist der trichterförmige Mantel mit wenigstens zwei von der unteren Öffnung kleinen Durchmessers zur oberen Öffnung großen Durchmessers verlaufenden Faltlinien versehen. Entlang dieser Faltlinien kann somit die Urinaltüte gefaltet und deren Außenkontur verringert werden.

In bevorzugter Ausführung der Erfindung besteht die Urinaltüte aus Papier, Karton oder Pappe. Weiterhin ist bevorzugt, daß diese aus zumindest einseitig gewachstem Papier, Karton oder Pappe besteht. Des weiteren kann diese aus Folie, einem folienartigen Material, insbesondere wasserfestem Material bestehen. Dadurch wird ein Aufweichen und Verformen der Urinaltüte durch die Urinflüssigkeit verhindert.

Es versteht sich, daß die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird im folgenden anhand eines Ausführungsbeispieles unter Bezugnahme auf die zugehörigen Zeichnungen weiter beschrieben. In den Zeichnungen zeigt:
- Fig. 1: eine Perspektivansicht einer erfindungsgemäßen Urinaltüte und
- Fig. 2: eine Seitenansicht der Urinaltüte nach Fig 1.

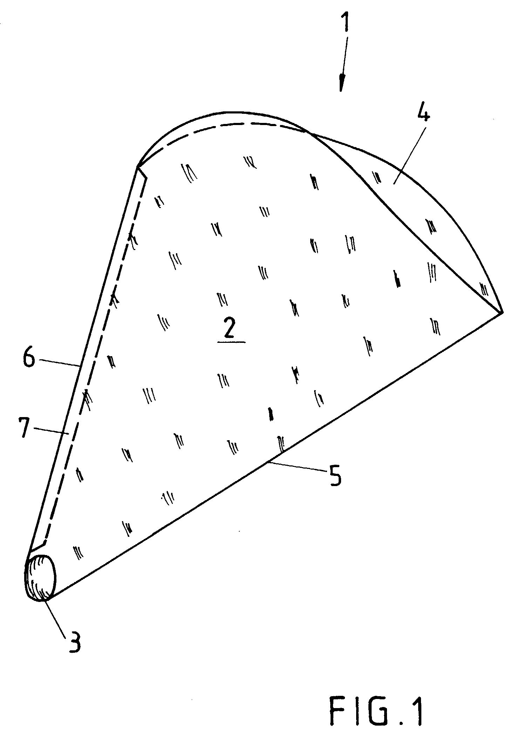

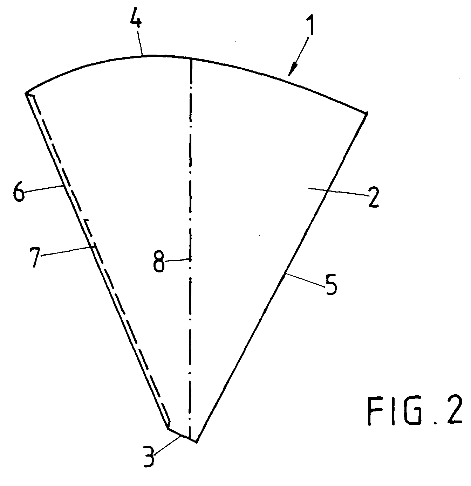

Die erfindungsgemäße Urinaltüte 1 besteht gemäß den Fig. 1 und 2 im kollabierten Zustand im wesentlichen aus einem trichterförmigen Mantel 2 aus Papier, Karton oder Pappe. Das Material soll eine Stärke aufweisen, die insbesondere geeignet ist, die Urinaltüte 1 aus ihrem kollabierten Zustand gemäß Fig. 2 in den aufgerichteten Zustand gemäß Fig. 1 zu überführen. Zweckmäßigerweise ist die Innen- oder Außenseite des Materials gewachst, um ein Durchweichen durch die Urinflüssigkeit zu vermeiden. Darüber hinaus darf das Material während des Gebrauchs der Urinaltüte nicht knittern oder sich verformen.

Der trichterförmige Mantel 2 weist im kollabierten Zustand nach Fig. 2 eine im wesentlichen dreieckige Form auf. Im aufgerichteten Zustand der Urinaltüte 1 nach Fig. 1 besitzt der trichterförmige Mantel 2 eine im allgemeinen Kegelform, wobei die Spitze des Kegels abgeschnitten ist. Dadurch wird eine untere Öffnung 3 kleinen Durchmessers erzeugt, durch welche die Urinflüssigkeit abgeleitet wird. Am der unteren Öffnung 3 kleinen Durchmessers gegenüberliegenden Seite des trichterförmigen Mantels 2 ist dieser mit einer oberen Öffnung 4 großen Durchmessers versehen. Wie insbesondere aus Fig. 2 ersichtlich ist, weist der trichterförmige Mantel 2 an seiner Außenkontur im Bereich der oberen Öffnung 4 einen Krümmungsradius auf, der sich von einer Seite 5 zur anderen Seite 6 des trichterförmigen Mantels 2 hin kontinuierlich verjüngt. Der stärker gekrümmte Bereich der oberen Öffnung 4 großen Durchmessers kann somit besser im Genitalbereich zwischen den Beinen des Benutzers angelegt werden, um den Genitalbereich möglichst dicht zu umschließen.

Entlang einer Seite 6 des trichterförmigen Mantels 2 ist zwischen der unteren Öffnung 3 kleinen Durchmessers und der oberen Öffnung 4 großen Durchmessers in den Mantel 2 ein Klebstreifen 7 integriert, um den Mantel 2 trichterförmig bzw. kegelförmig auszubilden. Zweckmäßigerweise ist die Außenseite des Klebstreifens 7 mittels einem geeigneten Klebstoff mit dem angrenzenden Bereich der Innenseite des Mantels 2 verklebt.

Zwischen der unteren Öffnung 3 kleinen Durchmessers und der oberen Öffnung 4 großen Durchmessers ist weiterhin, dargestellt durch eine Strich-Punkt-Strich-Linie, eine Faltlinie vorgesehen, um welche der Mantel 2 der Urinaltüte 1 gefaltet werden kann. Dadurch wird diese in ihrer Breite halbiert.

## Patentansprüche

1. Urinaltüte, insbesondere für Personen weiblichen Geschlechts, zum Anlegen am Genitalbereich der Person zum Zwecke des Urinierens, die aus einer im kollabierten Zustand im wesentlichen dreieckigen Form in ein trichterartiges Gebilde mit einem Mantel (2) mit einer oberen Öffnung (4) großen Durchmessers und eine dieser gegenüberliegende unteren Öffnung (3) kleinen Durchmessers aufrichtbar ist, dadurch gekennzeichnet, daß die Außenkontur des Mantels (2) im kollabierten Zustand der Urinaltüte im Bereich der oberen Öffnung (4) großen Durchmessers einen sich von einer Seite (5) zur anderen Seite (6) der im wesentlichen dreieckigen Form des Mantels (2), die sich jeweils von der unteren Öffnung (3) kleinen Durchmessers erstrecken, kontinuierlich verjüngenden Krümmungsradius aufweist, wobei die obere Öffnung (4) des Mantels (2) derart im Genitalbereich der Person anlegbar ist, daß dieser Bereich dicht umschlossen wird.

2. Urinaltüte nach Anspruch 1, dadurch gekennzeichnet, daß in deren kollabiertem Zustand die untere Öffnung (3) kleinen Durchmessers abgeschrägt ist.

3. Urinaltüte nach Anspruch 1 oder 2, dadurch gekennzeichnet, daß die untere Öffnung (3) einen Durchmesser von wenigstens 5 mm aufweist.

4. Urinaltüte nach einem der Ansprüche 1 bis 3, dadurch gekennzeichnet, daß die obere Öffnung (4) einen Durchmesser von wenigstens 50 mm aufweist.

5. Urinaltüte nach einem der Ansprüche 1 bis 4, dadurch gekennzeichnet, daß diese einen die obere Öffnung (4) großen Durchmessers und die untere Öffnung (3) kleinen Durchmessers verbindenden Klebstreifen (7) entlang des trichterförmigen Mantels (2) aufweist.

6. Urinaltüte nach einem der Ansprüche 1 bis 5, dadurch gekennzeichnet, daß der Klebstreifen (7) am trichterförmigen Mantel (2) verklebbar ist.

7. Urinaltüte nach Anspruch 1, dadurch gekennzeichnet, daß der Mantel (2) an zumindest einer seiner Seiten (5,6) durch Rändeln oder dergleichen verbunden ist.

8. Urinaltüte nach einem der Ansprüche 1 bis 7, dadurch gekennzeichnet, daß der trichterförmige Mantel (2) mit wenigstens zwei von der unteren Öffnung (3) kleinen Durchmessers zur oberen Öffnung (4) großen Durchmessers verlaufenden Faltlinien (8) versehen ist.

9. Urinaltüte nach einem der Ansprüche 1 bis 8, dadurch gekennzeichnet, daß die Urinflüssigkeit über die Öffnung (3) kleinen Durchmessers des Mantels (2) ableitbar ist.

10. Urinaltüte nach einem der Ansprüche 1 bis 9, dadurch gekennzeichnet, daß diese aus Papier, Karton oder Pappe besteht.

11. Urinaltüte nach einem der Ansprüche 1 bis 10, dadurch gekennzeichnet, daß diese aus Folie, einem folienartigen Material, insbesondere wasserfestem Material besteht.

## Claims

1. A urinal cone, particularly for persons of the female gender, to apply to the genital region of the person for the purpose of urinating, which urinal cone can be set up from an essentially triangular shape in the collapsed state to form a funnel-shaped body, comprising an envelope (2) with an upper opening (4) of large diameter and an opposite-positioned lower opening (3) of small diameter, characterized in that the outside contour of envelope (2) in the collapsed state of the urinal cone has in the region of the upper opening (4) of large diameter a continuously tapered radius of curvature, extending from one side (5) to the other side (6) of the essentially triangular shape of envelope (2), which sides extend respectively from the lower opening (3) of small diameter, wherein the upper opening (4) of envelope (2) can be applied to the genital area of the person in such a way that this area is enclosed tightly.

2. A urinal cone according to claim 1, characterized in that in the collapsed state of the urinal cone, the lower opening (3) of small diameter is beveled.

3. A urinal cone according to claim 1 or 2, characterized in that the lower opening (3) has a diameter of at least 5 mm.

4. A urinal cone according to one of the claims 1 to 3, characterized in that the upper opening (4) has a diameter of at least 50 mm.

5. A urinal cone according to one of the claims 1 to 4, characterized in that it has an adhesive strip (7) that connects the upper opening (4) at large diameter with the lower opening (3) of small diameter along the funnel-shaped envelope (2).

6. A urinal cone according to one of the claims 1 to 5, characterized in that the adhesive strip (7) can be glued to the funnel-shaped envelope (2).

7. A urinal cone according to claim 1, characterized in that the envelope (2) is connected on at least one of its sides (5, 6) through reeding or the like.

8. A urinal cone according to one of the claims 1 to 7, characterized in that the funnel-shaped envelope (2) has at least two foldinq lines (8) that extend from the lower opening (3) of small diameter to the upper opening (4) of large diameter.

9. A urinal cone according to one of the claims 1 to 8, characterized in that the urinary fluid can he discharged through the opening (3) of small diameter in envelope (2).

10. A urinal cone according to one of the claims 1 to 9, characterized in that it consists of paper or cardboard.

11. A urinal cone according to one of the claims 1 to 10, characterized in that this cone consists of a foil, a foil-type material, in particular a water-proof material.

## Revendications

1. La poche urinaire, spécialement conçue pour les personnes du sexe féminin, sera appliquée aux parties génitales en but d'uriner, lorsqu'elle est pliée, elle a sensiblement la forme d'un triangle que l'on peut déplier et qui se présente en forme d'entonnoir avec un manteau (2) ayant une ouverture supérieure (4) à grand diamètre et à l'opposé, l'ouverture inférieure (3) à petit diamètre, elle est caractérisée comme suit, lorsque la poche urinaire est pliée, le contour extérieur du manteau (2) allant de l'ouverture supérieure (4) de grand diamètre, d'un coté (5) à l'autre (6) de la forme essentiellement triangulaire du manteau (2) et conduisant respectivement jusqu'à l'ouverture inférieure (3) de petit diamètre, présentant ainsi un rayon de raccordement dégressif, si bien que l'ouverture supérieure (4) du manteau (2) peut, de telle manière, s'appliquer correctement à la partie génitale de la personne, cette application doit être étanche.

2. Poche urinaire selon exigence 1, est caractérisée par une ouverture inférieure (3) de petit diamètre et en biais lorsque la poche est pliée.

3. Poche urinaire selon exigence 1 ou 2, est caractérisée par une ouverture inférieure (3) qui présente un diamètre minimum de 5 mm.

4. Poche urinaire selon une des exigences de 1 à 3, est caractérisée par une ouverture supérieure (4) présentant un diamètre minimum de 50 mm.

5. Poche urinaire selon une des exigences de 1 à 4, est caractérisée par une bande adhésive (7) le long du manteau en forme d'entonnoir (2) qui relie l'ouverture supérieure (4) de grand diamètre et l'ouverture inférieure (3) de petit diamètre.

6. Poche urinaire selon une des exigences de 1 à 5, est caractérisée par une bande adhésive (7) collée le long du manteau en forme d'entonnoir.

7. Poche urinaire selon l'exigence 1, est caractérisée par une bande renforcée ou un assemblage semblable au moins d'un côté (5,6) du manteau (2).

8. Poche urinaire selon une des exigences de 1 à 7, est caractérisée par le fait que le manteau en forme d'entonnoir (2) soit muni de 2 lignes continues de pliage (8) allant de l'ouverture inférieure (3) à petit diamètre à l'ouverture supérieure (4) de grand diamètre (4)

9. Poche urinaire selon une des exigences de 1 à 8, est caracterisée par l'écoulement du liquide urinaire qui s'effectue par l'ouverture (3) de petit diamètre du manteau (2) .

10. Poche urinaire selon une des exigences de 1 à 10, est caractérisée par le fait qu'elle soit concue en papier, carton ou papier cartonné.

11. Poche urinaire selon une des exigences de 1 à 10, est caractérisée par le fait qu'elle est soit conçue en papier ou un matériel semblable, mais un matériel essentiellement étanche.
